(19) Europäisches Patentamt
European Patent Office
Office européen des brevets

(11) Publication number: **0 125 555**
**B1**

(12) **EUROPEAN PATENT SPECIFICATION**

(45) Date of publication of patent specification: **20.12.89**

(51) Int. Cl.[4]: **G 01 N 33/52**, G 01 N 33/84, C 07 C 119/00

(21) Application number: **84104828.3**

(22) Date of filing: **30.04.84**

(54) **Unified test means for ion determination.**

(30) Priority: **12.05.83 US 493983**

(43) Date of publication of application:
**21.11.84 Bulletin 84/47**

(45) Publication of the grant of the patent:
**20.12.89 Bulletin 89/51**

(84) Designated Contracting States:
**AT BE CH DE FR GB IT LI LU NL SE**

(56) References cited:
**EP-A-0 010 615**
**EP-A-0 041 175**
**EP-A-0 085 320**
**US-A-3 856 649**
**US-A-4 234 313**
**US-A-4 272 485**

**CLINICAL CHEMISTRY, volume 28, no. 9, September 1982, pages 1857-1861, Washington, US; S.C. CHARLTON et al.: "Solid-phase colorimetric determination of potassium"**

(73) Proprietor: **MILES INC.**
**1127 Myrtle Street**
**Elkhart Indiana 46514 (US)**

(72) Inventor: **Charlton, Steven C.**
**1533 Strong Avenue**
**Elkhart, IN 46514 (US)**
Inventor: **Fleming, Roger L.**
**3107 May St.**
**Niles, MI 49120 (US)**
Inventor: **Lau, Arthur L. Y.**
**2321 Kenilworth**
**Elkhart, IN 46514 (US)**
Inventor: **Hemmes, Paul**
**1825 Brookwood Drive**
**Elkhart, IN 46514 (US)**

(74) Representative: **Adrian, Albert, Dr. et al**
**c/o BAYER AG Konzernverwaltung RP**
**Patentabteilung**
**D-5090 Leverkusen 1, Bayerwerk (DE)**

**EP 0 125 555 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to the measurement of ions, in particular ions in aqueous solution, and to a test means or device for performing such measurements. The invention provides a quick, facile way of assaying such ions whereby results are available to the assayist momentarily after merely contacting a test sample solution with the test means or device. There is no need for cumbersome, expensive electronic equipment such as ion-specific electrodes, flame photometers, atomic absorption spectrophotometers or the like. Nor is it necessary to resort to time-consuming wet chemistry techniques such as titration and other laboratory procedures. The present invention enables the analyst to merely contact the test sample with a strip device or similar test means configuration, and observe any color change.

The determination of aqueous ion concentration has application in numerous technologies. In the water purification art, calcium concentration must be carefully monitored to assess the degree of saturation of an ion exchange resin deionizer. Measurement of sodium and other ions in seawater is important in the preparation of drinking water aboard a ship at sea. Measurement of the potassium level in blood aids the physician in diagnosis of conditions leading to muscle irritability and excitatory changes in myocardial function. Such conditions include oliguria, anuria, urinary obstruction and renal failure due to shock.

Needless to say, a quick, facile method for determining ion concentration would greatly enhance the state of these technologies, as well as any others where such rapid, accurate determinations would be beneficial. Thus, for example, if a medical laboratory technician could accurately measure the potassium or calcium level of a serum or whole blood sample in a matter of seconds or minutes, not only would such rapid results aid the physician in diagnosis, but also laboratory efficiency would increase manyfold.

Prior to the present invention, methods for determining ions in solution included flame photometry, atomic absorption photometry and ion-specific electrodes. The use of certain compounds and compositions which selectively isolate certain ions from the sample solution has become popular in ion-specific electrodes. These substances, known as ionophores, have the capability of selectively isolating ions from their counterions thereby causing a charge separation and a corresponding change in electrical conductivity in the phase containing the ionophore. Illustrative of the ion/ionophore phenomenon include ion assays utilizing membrane electrodes, liquid/liquid partitioning and fluorescence.

When two solutions having different concentrations of ions are separated by an electrically conductive membrane, an electrical potential (EMF) is generated. The EMF developed by such system is a function of concentration or ionic activity. This phenomenon is expressed mathematically by the well-known Nernst Equation

$$\varepsilon = \frac{RT}{nF} \ln \frac{\gamma_1 c_1}{\gamma_2 c_2} \qquad (1)$$

in which $\varepsilon$ is the EMF of the particular system, F is the Faraday Constant [23,062.4 $\pm$ 0.003 calories (volt equiv.)$^{-1}$], R is the gas constant, T is the temperature in °C and $\gamma$ and c are, respectively, the activity coefficients and molal concentrations of the ion under study, the subscript 1 designates the solution on one side of the membrane, the subscript 2 denoting the solution on the other side, and n is the number of electrons transferred in the reaction.

In such membrane separation cells, the membrane can be a simple fritted glass barrier, allowing a small but measurable degree of ion diffusion from one solution to the other. Alternatively, a nonporous, electrically nonconductive film, such as polyvinyl chloride, impregnated with an ionophore can be employed. In the absence of the ionophore the film is an insulator and no EMF can be measured; when blended with an ionophore, charged ions are bound to the film and a small, measurable current can be induced to flow. Because the ionophore is selective in its affinity, and thus will bind only certain specific ions, such cells are ion selective. Any measurable EMF is due solely to the presence of those ions.

Thus, a cell for determining potassium ions ($K^+$) can be produced through use of an ionophore specific for $K^+$, e.g. valinomycin. In the presence of potassium, valinomycin produces a conductive path across a membrane by binding and transporting $K^+$, thus allowing a small current to flow. A reference concentration of $K^+$ is placed on one side of the membrane and the test sample on the other. The EMF developed is measured and used to calculate the unknown concentration from equation (1). Because $K^+$ binds to the valinomycin membrane, the conductive path only appears for $K^+$. Therefore, the only EMF developed is attributable solely to the $K^+$ concentration gradient across the membrane.

The current flowing across the membrane is so small that no significant quantity of $K^+$ or counterion is transported through it. Electrical neutrality of the membrane is maintained either by a reverse flow of hydrogen ions, or by a parallel flow of $OH^-$. This anion effect can reduce the specificity of the electrode towards the intended ion and is an interference to be minimized.

A major difficulty in the use of such ion-selective electrodes has been the marked reduction of accuracy and speed of response over time. Further, small changes in ion concentration produce such small changes in EMF that sophisticated voltmeter equipment is required.

It has been known that certain antibiotics, such as valinomycin, have an effect on the electrical properties of phospholipid bilayer membranes (biological membranes), such that these antiobiotics solubilize cations within the membrane, in the form of mobile charged couples, thereby providing a

"carrier" mechanism by which cations can cross the insulating hydrocarbon interior of the membrane. Such complexes have the sole purpose of carrying the charge of the complex through the membrane such that a voltage differential can be determined between solutions on either side of the membrane.

CH—A—479 870 which is corresponding to DE—A—1648 978 describes the use of porous membranes impregnated with macrocyclic derivatives of amino and oxy-acids in ion-sensitive electrodes. Materials used to form the membrane are glass frits and other porous membranes. Such electrodes are said to be effective in measuring ion activities.

United States Patent No. 4,053,381, issued to Hamblen, *et al.* discloses similar technology, and utilizes an ion specific membrane having ion mobility across it.

Another known application of ionophores in ion determination is through liquid/liquid partitioning. In this procedure, a hydrophobic ionophore is dissolved in an organic solvent immiscible with water. Eisenman *et al.,* J. Membrane Biol. 1:294—345 (1969) disclose the selective extraction of cations from aqueous solutions into organic solvents via macrotetralide actin antibiotics. This technique involves merely shaking an organic solvent phase containing the antibiotics with aqueous solutions containing cationic salts of lipid-soluble colored anions, such as picrates and dinitrophenolates. The intensity of color of the organic phase is then measured spectrophotometrically to indicate how much salt has been extracted. Phase transfer has also been studied by Dix *et al., Angew, Chem. Int. Ed. Engl. 17:*857 (1978) and in reviews including Burgermeister *et al., Top. Curr. Chem. 69*:91 (1977) Yu *et al.*, "Membrane Active Complexones," Elsevier, Amsterdam (1974); and Duncan, "Calcium in Biological Systems," Cambridge University Press (1976).

Sumiyoshi, et al., *Talenta, 24,* 763—765 (1977) describes another method useful for determining $K^+$ in serum. In this technique serum is deproteinated by trichloroacetic acid, an indicator dye is added, and shaken with a solvent such as chloroform containing valinomycin.

Partitioning of a compound is rapid and effective between liquids, as shown by Eisenman, because of the mobility of the ionophore carrier and ions, which allows the transported species to diffuse rapidly away from the interface. Such a mechanism is normally impossible in the solid phase, because of the rigidity, immobility and essentially zero diffusion of materials in a solid phase.

Yet another approach to the measurement of ion activity in aqueous solutions utilizes fluorescent anions. Feinstein, *et al., Proc. Nat. Acad. Sci. U.S.A., 68,* 2037—2041 (1971). It is stated that the presence of cation/ionophore complexes in organic solvents are known, but that complex formation in purely aqueous media had theretofore not been detected. Feinstein, *et al.,* demonstrated the existence of such complexes in water through the use of the fluorescent salts 1-anilino-8-naphthalene sulfonate and 2-*p*-toluidinyl sulfonate.

It was found that interaction of the ionophore/cation complexes with the fluorescent dyes produced enhanced fluorescence emission, increased lifetime and polarization, and significant blue-shift at the emission maxima of the fluorescence spectrum. At constant concentrations of ionophore and fluorophore, the intensity of fluorescence emission was found to be a function of cation concentration.

An ion assay is disclosed in U.S. Patent No. 4,367,072 which makes use of a conjugate of an ionophore covalently bound to a chromophore material. In use, the conjugate is added to a liquid sample and the appearance of color in the solution is monitored spectrophotometrically.

The disclosure is limited to a solution assay, and it appears that insufficient color develops to enable direct visual observation. Moreover, the stoichiometric ratio of chromophore to ionophore is fixed in such a system due to the direct bonding between these molecules. Because of this direct bonding it is impossible to regulate color intensity; the ratio of ionophore to chromophore is fixed.

EP—A 241 175 describes a test device wherein only an ionophore is incorporated in a nonpolar matrix material. The dye is used as separated from the ionophore.

EP— 10 615 is dealing with chromogenic selective complexing ligands and host molecules which are covalently linked to a dye-stuff.

To summarize the background of technological developments leading up to the present invention, many methods are known for assaying ions in solution. Instrumental methods include such sophisticated techniques as ion-specific potentiometry, flame photometry and atomic absorption photometry. The use of ionophores which selectively complex with specific ions has led to four basic approaches: ion selective electrodes, liquid/liquid partitioning, fluorescence enhancement, and chromophore-labeled ionophore conjugates.

None of these approaches, however, affords the assayist simple, fast analysis results through contacting a test sample solution with a test means or device. The present invention, on the other hand, permits the assayist to merely contact the sample with a dip-and-read test strip or device of similar configuration, and observe a change in color or other detectable response. Moreover, the degree of such response can be regulated by varying the stoichiometry of the reactants which produce it.

Figures 1—7 are graphical representations of the data obtained in Examples 2, 8, respectively,

In Figure 1 the detection of potassium using naphtho-15-crown-5 as the ionophore in Example 2 is shown.

Figure 2 is a graphical representation of the data obtained in Example 3 in which both naphtho-15-crown-5 and valinomycin are present in the test means as ionophores.

Figure 3 depicts the results of Example 4 in which equal amounts of naphtho-15-crown-5 and

valinomycin are utilized in the test means.

Figure 4 shows the results of using only valinomycin as the ionophore in a potassium test means, as described in Example 5.

Figure 5 shows the results of using dipentyl phthalate as a plasticizer in the test means of Example 6.

Data from Example 7, in which a test means responsive to sodium ion concentration is described, is portrayed graphically in Figure 6.

Figure 7 is a plot of the data obtained in Example 8, in which the reporter substance is tetrabromophenolphthalein ethyl ester.

The present invention resides in the discovery of a new test means for detecting the presence of a specific ion in an aqueous test sample and to determining its concentration. The test means comprises a substantially nonpolar, nonporous carrier matrix which is incorporated with an ionophore capable of selectively forming a complex with the ion under analysis. In addition, the carrier matrix is incorporated with a reporter substance which is capable of producing a detectable response, such as a change in or appearance of color or fluorescence.

A test device which utilizes the test means comprises an elongated support member, such as a plastic film, to one flat side of which is affixed the test means.

In use the sample is contacted with the test means or device, and the presence and/or concentration of the ion is then determined by observing any detectable response produced.

The test means and device of the present invention provide rapid results, sufficient detectable response forming in most instances in at least a few minutes. No cumbersome, expensive testing equipment is required in addition to the present invention. Moreover, it has been found that the color or other response produced in the test means is stable, in some instances for a period of days, such that a number of used test means can be set aside for reading at some future time.

Certain terms used in the present discussion should at this point be mentioned to assure that the reader is of the same mind as the author as to their respective meanings. Thus the following definitions are provided to clarify the scope of the present invention, and to enable its formulation and use.

The term "ionophore" includes molecules capable of selectively forming a complex with a particular ion to the substantial exclusion of others. For example the cyclic polypeptide valinomycin, binds selectively to potassium ions in solution to form a cationic complex. Also included in the term are crown ethers, cryptands and podands.

As used herein, "substantially nonpolar" is intended as meaning that quality of a substance not to exhibit a substantial dipole moment or electrical polarity. In particular, it includes non-ionic substances, and substances which are dielectric.

The term "nonporous" is intended to mean substantially impervious to the flow of water. Thus a nonporous carrier matrix is one which precludes the passage of water through it, one side to the other. For example, a polyvinyl chloride film would be considered for the purposes herein as being non-porous.

A "reporter substance" is one which is capable of interacting with an ionophore/ion complex to produce a color change or other detectable response. Thus, the reporter can be an ionic dye such that when the dye is in its ionized state it is a counterion, i.e., opposite in charge, to the ion to be analyzed. Some examples of these are Erythrosin B, 7-amino-4-trifluoromethyl coumarin and 2,6-dichloroindophenol sodium salt. The reporter also includes phenolic compounds such as p-nitrophenol, which are relatively colorless in the non-ionized state, but which color upon ionization. The reporter substance can also be one which can trigger a detectable response together with other components. For example, the iodide ion can produce a detectable response by interacting with the ionophore/ion complex in the presence of starch and an oxidizing agent.

By "interacting" is meant any coaction between a reporter substance and an ionophore/ion complex which leads to a detectable response. An example of the reporter substance interacting with the complex is in the case where the reporter is changed by the complex from a colorless to a colored state, such as in the case of p-nitrophenol.

The term "detectable response" is meant herein as a change in or occurrence of a parameter in a test means system which is capable of being perceived, either by direct observation or instrumentally, and which is a function of the presence of a specific ion in an aqueous test sample. Some detectable responses are the change in or appearance of color, fluorescence, reflectance, pH, chemiluminescence and infrared spectra.

By the term "intermediate alkyl" as used herein is meant an alkyl group having from about 4 to about 12 carbon atoms. It includes normal and branched isomers. It may be unsubstituted or it may be substituted, provided any such substitution not interfere with the operation of the presently claimed test means or device in its capability to detect ions.

The term "lower alkyl", as used in the present disclosure, is meant an alkyl moiety containing about 1—4 carbon atoms. Included in the meaning of lower alkyl are methyl, ethyl, *n*-propyl, isopropyl, *n*-butyl, *sec*-butyl and *tert*-butyl. These may be unsubstituted, or they may be substituted provided any such substituents not interfering with the operation or functioning of the presently claimed test means or device in its capability to detect ions.

By "pseudohalogen" is meant atoms or groups of atoms which, when attached to an unsaturated or aromatic ring system, affect the electrophilicity or nucleophilicity of the ring system, and/or have an ability

to distribute an electrical change through delocalization or resonance, in a fashion similar to the halogens. Thus, whereas halogen signifies Group VII atoms such as F, Cl, and I, pseudohalogens embrace such moieties as $-CN$, $-SCN$, $-N_3$, $-COR$, $-COOR$, $-CONHR$, $-CF_3$, $-CCl_3$, $-NO_2$, $-SO_2CF_3$, $-SO_2CH_3$, and $-SO_2C_6H_4CH_3$, in which R is alkyl or aryl.

The present test means comprises three basic elements: a substantially nonpolar, nonporous carrier matrix; an ionophore; and a reporter substance. When an aqueous test sample contains an ion capable of specifically complexing with the ionophore, the ion can then enter the matrix and interact with the reporter substance, thereby producing a detectable response.

In order for the test means to provide a detectable response solely as a result of the presence of a specific ion, it is necessary that other ions be substantially excluded from entering the carrier matrix. This is because it is the ionophore/ion complex which triggers the detectable response in conjunction with the reporter substance. Accordingly, the carrier matrix must be fabricated from a material which is both nonpolar and nonporous. Exemplary of such materials are films of such polymers as polyvinyl fluoride, polyvinyl chloride, vinyl chloride/vinyl acetate copolymer, vinyl chloride/vinylidene chloride copolymer, vinyl chloride/vinyl acetate/vinyl alcohol terpolymer, vinylidene chloride/acrylonitrile copolymer, and polyurethane. Of course, many other polymeric materials would be suitable for use in the present invention, and the identification of such materials would be well within the skill of the art, given the present disclosure.

Other, nonpolymeric, materials would include ceramic substances, a painted substance (in which the paint layer would be the carrier matrix), glass-like substances, and other nonpolar materials.

It is required that the carrier matrix is non-porous and nonpolar, because the ion must not be able to substantially penetrate the matrix unless it is that particular ion or ions for which the ionophore has complexing affinity. The concept of a nonporous matrix, of course, does not exclude microscopic porosity. It is clear from the foregoing remarks as well as the very nature of the invention, that some porosity could be possible provided the analyte ion be precluded from permeation of the carrier matrix to a sufficient degree to cause the detectable response to occur, absent the presence of the ionophore.

The composition of the carrier matrix in the present invention is to be carefully distinguished over prior art test means whereby porous materials such as paper were used. In that type of device, it is required that any test sample to which the device is exposed be capable of permeating the entire reagent area. Such test devices function on entirely different principles from the present one, and a paper carrier matrix is not considered as within the scope of the present invention unless such paper matrix be rendered substantially nonpolar and nonporous, i.e., such as by polymer or wax impregnation.

Thus, the carrier matrix is one which is not wetted by water, i.e., one which precludes substantial penetration by the aqueous test sample. Moreover, it is intended that both the ionophore and reporter substance become virtually insoluble in the aqueous test sample due to their being entrapped with the carrier matrix. The requirement of nonporosity of the carrier matrix is to preclude dissolution or leaching of ionophore or the reporter substance, as well as to prevent permeation by test sample components other than the ionic analyte.

The ionophore element of the present invention is indeed a concept which is broad in scope, as characterized by the definition of the term as given above. It includes multidentate cyclic compounds which contain donor atoms in their cyclic chains. Such multidenate cyclic compounds can be monocyclic or polycyclic. Alternatively, the ionophore can be an open chain containing donor atoms. Thus, included in the term are monocyclic systems which are ion-specific, termed coronands; polycyclic ion-specific compounds known as cryptands; and open chain structures, known as podands, which are capable of selectively complexing with ions.

The coronands are monocyclic compounds which contain donor atoms which are electron rich or deficient and which are capable of complexing with particular cations and anions because of their unique structures. Included in this term are the crown ethers in which the monocyclic chain contains oxygen as the donor atoms. Other coronands are compounds which contain an assortment of electron rich atoms such as oxygen, sulfur and nitrogen. Because of the unique sizes and geometries of particular coronands, they are adaptable to complexing with various ions. In so complexing, the electron rich atoms, such as the oxygens in a crown ether, orient towards the electron deficient cation. The carbon atom segments of the chain are simultaneously projected in a direction outwards from the ion. Thus, the resultant complex is charged in the center, but is hydrophobic at its perimeter.

The cryptands are the polycyclic analogues of the coronands. Accordingly, they include bicyclic and tricyclic multidentate compounds. In the cryptands, the cyclic arrangement of donor atoms is three dimensional in space, as opposed to the substantially planar configuration of the coronand. A cryptand is capable of virtually enveloping the ion in three dimensional fashion and, hence, is capable of strong bonds to the ion in forming the complex. Like in the coronands, the donor atoms can include such atoms as oxygen, nitrogen and sulfur.

Ions can also be selectively complexed with noncyclic compounds. For example, a linear chain which contains a regular sequence of electron rich atoms such as oxygen has the capability of associating with positively charged ions to form complexes, not entirely unlike the coronands and cryptands. The main structural difference between podands and the other two ionophores is the openness of the structure. Thus, podands can be subcategorized into monopodands, dipodands, tripodands, . . . A monopodand,

therefore, is a single organic chain containing donor atoms, a dipodand is two such chains attached to a central atom or group of atoms, and is capable of variable special orientation, and a tripodand is three such chains.

Some of the ionophores which have been found to be especially useful with the instant invention are tabulated herein along with the cations with which they are capable of selectively complexing.

| Ionophore | Cation |
|---|---|
| Valinomycin | $K^+$ |
| 4,7,13,16,21-Pentaoxa-1,10-diazabicyclo-[8,8,5]tricosane (Kryptofix 221) | $Na^+$ |
| 4,7,13,16,21,24-Hexaoxa-1,10-diazabicyclo-[8,8,8]hexacosane (Kryptofix 222) | $K^+$ |
| 4,7,13,18-Tetraoxa-1,10-diazabicyclo-[8,5,5]eicosane (Kryptofix 211) | $Li^+$ |
| 12-Crown-4 | $Li^+$ |
| 15-Crown-5 | $Na^+$, $K^+$ |
| 18-Crown-6 | $K^+$ |
| Dibenzo-18-crown-6 | $K^+$ |
| Dicyclohexano-18-crown-6 | $K^+$ |

Kryptofix is a registered trademark of E. Merck, Darmstadt, Germany

Given the presence of the ion of interest in the test solution, it is the reporter substance which provides the detectable response as a result of its interacting with the ionophore/ion complex. The reporter substance can range in composition from a single compound, such as chromogenic counterion, to a mixture of reactive species which produce a detectable product when their reaction chain is triggered by the complex. Thus, it can be seen that when no analyte ion is present the reporter substance remains dormant; no detectable response is observed. Alternatively, when the particular ion under surveillance is present, it is enabled by the ionophore to enter the carrier matrix to form a complex, which complex interacts with the reporter substance and induces it to undergo a detectable change.

In the case where the reporter is a single compound, it can include a salt or other dissociable compound, such that upon dissociation a colored ionic species is formed. Depending on the charge of the analyte ion, an ionic reporter is chosen such that the colored ion is opposite in charge to the analyte. Also useful is a dissociable compound in which the counterion to the analyte is fluorescent. Examples of such chromophoric and fluorophoric reporter substances are dichlorophenolindophenol, fluorescein and its derivatives, 8-anilino-1-naphthalenesulfonic acid, 7-amino-4-trifluoromethyl coumarin, Erythrosin B, Orange IV, Phloxine B, and Eosin Y. Structures of Erythrosin B, Phloxine B and Eosin Y are given in "Aldrich Handbook of Fine Chemicals", Aldrich Chemical Company, Milwaukee (1983). The structure of Orange IV can be found in "The Merck Index", 9th ed., Merck & Co., Inc. Rahway (1976).

Where the reporter substance comprises a mixture of reactive species as at least two precursor substances which are capable of participating in the formation of a detectable response in the presence of the complex of the ionophore and the ion, great latitutude is possible in selecting an appropriate combination of ingredients. For example, one system could be iodide ion, starch and an oxidizing agent. Such a system could be utilized in a test means in which the carrier matrix contains (in addition to ionophore) starch and the oxidizer. Iodide could then be added to the test sample. In the presence of an analyte ion, formation of the ion/ionophore complex would induce iodide to associate with the matrix, whereupon it would be converted to free iodine, thus indicating a positive test.

Yet abother example of a reaction sequence useful as the reporter substance is one which involves the dissociation of a proton from a phenol, thus initiating a coupling reaction to form a colored product. The so-called Gibbs Reaction is typical of such a reaction sequence, in which 2,5-cyclohexadiene-1-one-2,6-dihalo-4-haloimine couples with a phenol to form a colored reaction product

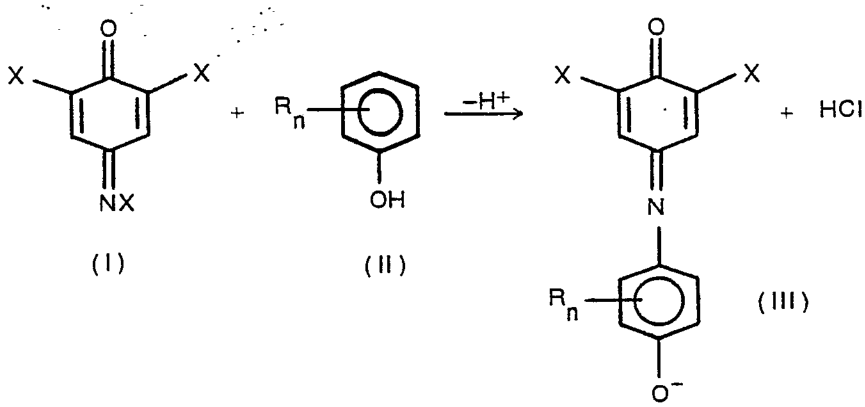


(I) (II) (III)

In this reaction sequence R can be any 2, 3, 5, and/or 6 position substituent which will not hinder the overall reaction sequence. Thus R is H, lower or intermediate alkyl or aryl, or R can form a fused ring system at either the 2,3- or 5,6-positions. X is halogen such as F, Cl, Br and I, or X can be a pseudohalogen and n is 0—4. This kind of reporter substance can be utilized by incorporating compounds having the structures (I) and (II) directly with the carrier matrix.

Still another utilization of the Gibbs chemistry involves compounds having a structure such as (III) in its nonionized form. The formation of the ion/ionophore complex results in an interaction such that reporter substance (III) yields observable color in and of itself. This phenomenon can be thought of as proceeding in accordance with

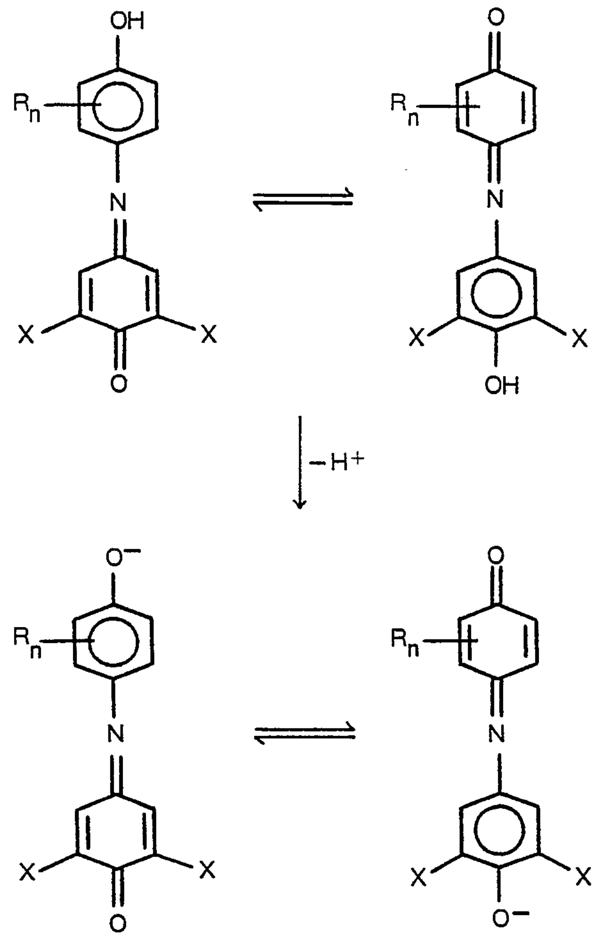


in which R is lower alkyl or aryl, or any two of R together form a fused ring system, *n* is 0—4 and X is as defined above. Especially preferred is a compound having the structure

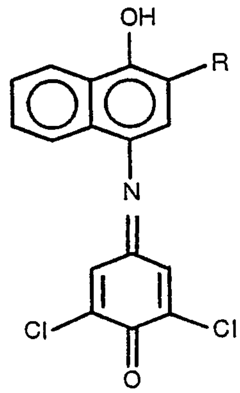

Yet another preferred reporter substance is a compound having the structure

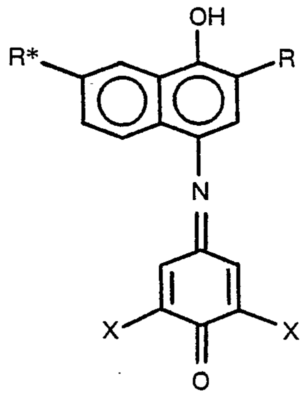

in which R* is an intermediate alkyl group, i.e., having 4—12 carbon atoms, R is H or $C_1$ to $C_4$ lower alkyl and X is a halogen or pseudohalogen, especially Cl. Compounds such as these have been found to be especially resistant to possible interference due to the presence of serum albumin in the test sample. Preferred among these types of reporter substances is that in which R* is *n*-decyl and R is methyl. Tetrabromophenolphthalein alkyl esters have also been found to be preferred reporter substances.

The test means described above can be used by itself or it can be mounted at one end of an elongated support member, the other end serving as a handle. Such a test device can be held at the handle end, while the other end bearing the test means is contacted with the test sample.

Useful materials for the support member include films of a myriad of plastics or polymers. Examples include such polymeric materials as cellulose acetate, polyethylene terephthalate, polycarbonates and polystryene. The support can be opaque or it can transmit light or other energy. Preferred supports include transparent materials capable of transmitting electromagnetic radiation of a wavelength in the range of about 200 nanometers (nm) to 900 nm. The support need not, of course, transmit over the entire 200—900 nm region, although for fluorometric detection of analytical results it is desirable that the support be transparent over a band wider than, or at least equal to the absorption and emission spectra of the fluorescent materials used for detection. It may also be desirable to have a support that transmits one or more narrow wavelength bands and is opaque to adjacent wavelength bands. This could be accomplished, for example, by impregnating or coating the support with one or more colorants having suitable absorption characteristics.

To prepare a test device of the present invention, a small rectangle of the test means, i.e., a carrier matrix incorporated with an ionophore, a reporter substance and possibly other ingredients, is affixed to an elongated support member having an upper substantially flat face, such as an oblong piece of polystyrene film. The test means piece is affixed to the flat face at one end, leaving the other end of the polystyrene to serve as a convenient handle.

The test means can be affixed by any means compatible with the intended use. A preferred method is by using a double faced adhesive tape between the test means square and the support member. One such tape is known as Double Stick®, available from 3M Company.

The test means and device of the present invention can be adapted for use in carrying out a wide variety of chemical analyses, not only in the field of clinical chemistry, but in chemical research and chemical process control laboratories. They are well suited for use in clinical testing of body fluids such as blood, blood serum and urine, since in this work a large number of repetitive tests are frequently conducted, and test results are often needed a very short time after the sample is taken. In the field of blood analysis, for example, the invention can be adapted for use in carrying out quantitative analysis for many of the ionic blood components of clinical interest.

The test means (and test device) is used by contacting it with the test sample, and observing a detectable response. If the ion under analysis is present in the test sample, the complex of ionophore and ion will interact with the reporter substance and a detectable response will appear. Where the reporter substance, for example, is a dissociable salt producing a colored counterion of the analyte, the observable response will be the appearance of or change in color in the test means. Where the reporter substance is a fluorophore such as fluoroscein, a fluorescence spectrophotometer can be utilized to measure the detectable response formed in the test means (here, the appearance of or change in fluorescence). Other techniques useful in observing a detectable response include reflectance spectrophotometry, absorption spectrophotometry and light transmission measurements.

When the test sample is blood serum, transmission techniques can be used to detect and quantify the presence of any reaction products, the formation of which serves as the detectable reponse. In this case radiant energy such as ultraviolet, visible or infrared radiation, is directed onto one surface of the test means and the output of that energy from the opposite surface is measured. Generally, electromagnetic radiation in the range of from about 200 to about 900 nm has been found useful for such measurements, although any radiation permeating the test means and which is capable of signifying the occurrence or extent of the response can be used.

Various calibration techniques are applicable as a control for the analysis. For example, a sample of analyte standard solution can be applied to a separate test means as a comparison or to permit the use of differential measurements in the analysis.

## Examples

The following Examples are provided to further assist the reader in making and using the present invention. Thus, preferred embodiments are described in experimental detail and analyzed as to the results. The Examples are meant to be illustrative only, and are in no way intended as limiting the scope of the invention described and claimed herein.

### 1 Preparation of 7-(n-Decyl)-2-methyl-4-(3',5'-dichlorophen-4-one)-indonaphthol

The captioned compound (hereafter 7-decyl-MEDPIN) was prepared for use as a reporter substance in the present test means and test device. The reaction pathway is depicted in the following sequence, in which R* is *n*-decyl.

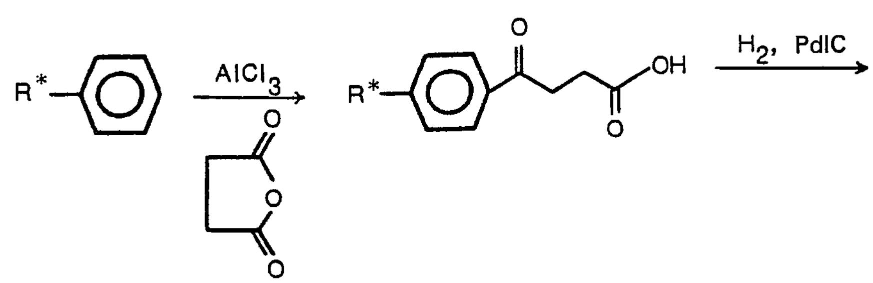


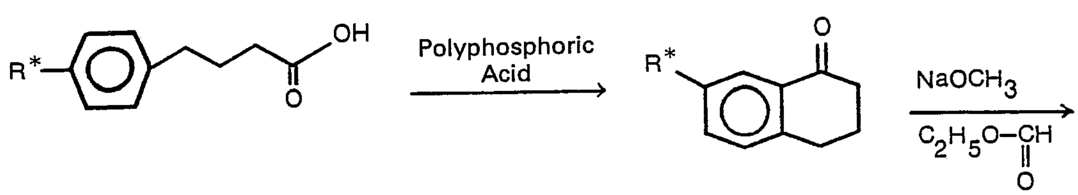


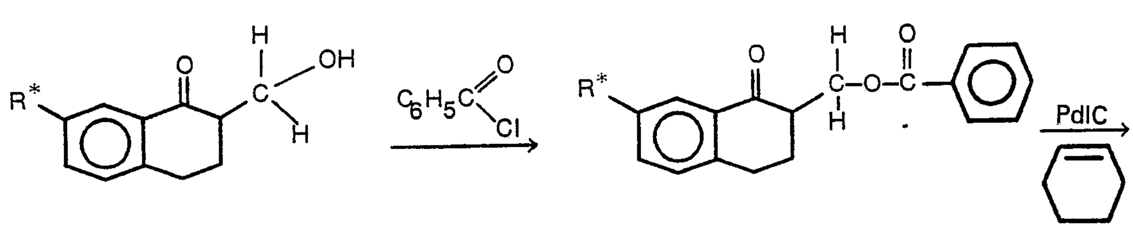

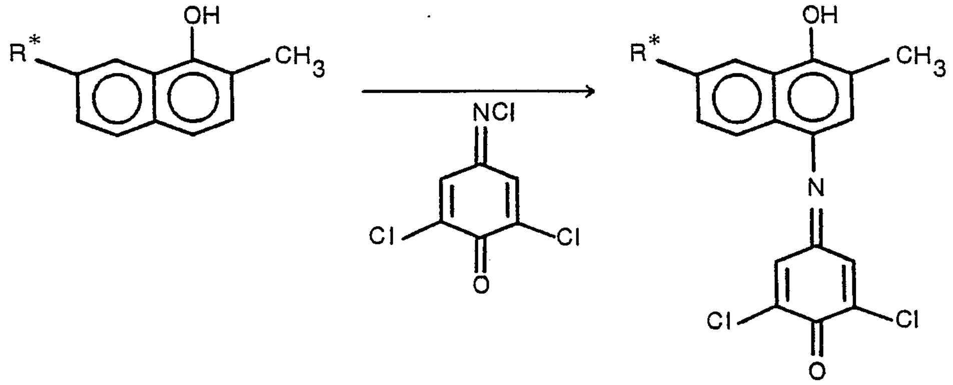

Preparation of β-(p-n-Decylbenzoyl)-propionic Acid

A mixture of 26.2 grams (g) phenyl-*n*-decane (1.2 mole), 120 g succinic anhydride (1.2 mole) and 360 mililiters (ml) nitroethane in a 5 liter (l) three-necked flask equipped with HCl outlet and mechanical stirrer was cooled to 0°C in an ice-bath. To this mixture 360 g $AlCl_3$ (2.7 moles) was added slowly over ½ hour with stirring. Evolution of HCl was observed when about half of the $AlCl_3$ was added. After the addition, the ice bath was removed, the reaction mixture was allowed to stand at room temperature (RT) for 5 minutes. The mixture was then heated over a steam bath. The heating and stirring was continued until the vigorous evaluation of HCl ceased (about 30 minutes). The reaction was cooled in an ice bath while 2 l of ice water was added followed by 600 ml of concentrated HCl. This was stirred at RT for 2 hours until all the dark brown solid was hydrolyzed. The insoluble product was recovered by filtration. The solid was then recrystallized twice with acetic acid (250 ml each time) to give about 320 g (85% yield) of product (dried in vacuum over KOH). TLC: Rf 0.43 in 1:1 (v/v) ethylacetate:toluene (silica gel plate).

Analysis: Calculated for $C_{20}H_{30}O_3$: C, 75.42; H, 9.50.
Found: C, 76.02; H, 9.89.

Preparation of 4-(p-n-decyl-phenyl)-butyric acid

Twenty grams of Pd/C (palladium-saturated carbon obtained from Aldrich Chemical Co., catalogue No. 20,569-9) and β-(*p-n*-decyl benzoyl)-propionic acid (150 g 0.47 moles) were mixed with acetic acid (350 ml) in a 1 l Paar bomb. The reaction was started at 3,43 × $10^5$ Pascal (50 psi (pounds per inch)) $H_2$ pressure and 50°C. A sudden increase in temperature accompanied by a drop in $H_2$ volume was observed. Thin layer chromatography reaction mixture indicated complete reaction. The catalyst was removed by glass fiber filtration while hot. The filtrate was allowed to crystallise at RT. The crystalline product was recovered by filtration. A second crop of product which formed after the filtrate was chilled was also recovered. The total yield was 100 g (68%) after drying under a vacuum over KOH. The melting point was 67—69°C.

TLC: Rf 0.68 in 1:1 (v/v) ethylacetate:toluene (silica gel plate).

Analysis: Calculated for: $C_{20}H_{32}O_2$: C, 78.90; H, 10.50.
Found: C, 78.39; H, 10.70.

Preparation of 7-n-Decyl-1-tetralone

A mixture of p-decyl-phenyl butyric acid (30 g, 98.7 mmoles) and polyphosphoric acid (150 g) was heated in an oil bath until all solid was melted. The heating was elevated to 150°C (internal temp.) and the mixture was stirred vigorously for 30 minutes. The reaction was then cooled to RT and treated with 300 ml ice water and 150 ml ethyl ether. After the mixture was stirred for 30 minutes at RT, the aqueous layer was separated and washed twice with 150 ml ethyl ether. The combined organic phases were washed with 150 ml saturated aqueous NaCl. Ether was removed by evaporation and the residue was distilled on a Kügelrohr distillatation apparatus (Aldrich Chemical Co.). The product had a boiling point of 190°—200°C 13,3 Pascal (≙0.1 mm Hg). The yield was 11 g (39%) of pale yellow oil.

TLC: Rf = 0.34 in toluene (silica gel plates).

Analysis Calculated for: $C_{20}H_{30}O$: C, 83.90; H, 10.70.
Found: C, 85.63; H, 10.83.

Preparation of 2-Hydroxymethylene-7-n-decyl-1-tetralone

A mixture of sodium methoxide (5.4 g 40.5 moles), ethyl formate (7.4 g, 100 mmoles) and 100 ml dry toluene was cooled in an ice bath under inert atmosphere ($N_2$). A solution of the 7-decyl-tetralone (11.5 g, 40 mmoles) in 100 ml dry toluene was added with rapid stirring. The ice bath was removed and the reaction was stirred at RT for 4 hours. The reaction mixture was treated with 100 ml water and 100 ml 6N HCl. The organic layer was separated and washed twice with 50 ml saturated NaCl, dried over anhydrous $Na_2SO_4$, filtered and evaporated to remove all the toluene. The oily residue was used for the next reaction without further purification.

TLC: Rf = 0.56 in toluene (silica gel plates), the spot turned dark-brown after spray with 5% $FeCl_3$ solution.

Preparation of 2-Benzoyloxymethylen-7-n-decyl-1-tetralone

The oily residue from the previous reaction step was mixed with dry pyridine (120 ml). The solution was stirred under nitrogen at 0°C (ice bath). The solution was treated with 30 ml of benzoyl chloride. After the addition of the benzoyl chloride, insoluble pyridinium chloride was observed in the mixture. The reaction was stirred at RT for two hours. The product was poured into ice water (400 ml) with vigorous stirring. The light cream color solid was recovered by filtration, and washed well with cold water. The slightly wet solid was recrystallized from hot absolute ethanol (120 ml). White solid (14 g, 87% yield based on the 7-decyl-1-tetralone) was recovered. The melting point was 64—66°C.

TLC: Rf = 0.40 in toluene (silica gel plates).

Analysis Calculated for: $C_{28}H_{34}O_3$: C, 80.34; H, 8.19.
Found: C, 80.05; H, 8.27.

Preparation of 7-n-Decyl-2-methyl-1-naphthol

To a mixture of 2-benzoyloxymethylene-7-(n-decyl)-1-tetralone (14 g, 33.5 mmoles) and PD/C (3.5 g) under inert atmosphere was added cyclohexane (175 ml). The mixture was heated to reflux while maintaining the inert atmosphere. The conversion of starting material to product was determined by TLC after 3 hours. After all the starting material reacted, the mixture was cooled down to RT. The catalyst was removed by filtration and washed twice with 50 ml hot toluene. The combined filtrate was evaporated to a small volume. The product was purified with a Prep-500 silica gel column (a high pressure silica gel preparative column, obtained from Waters Association, Milford, MA). Toluene was used as the mobile phase. The product fractions were pooled and evaporated to dryness under vacuum overnight. Cream white solid (0.0 g 90% yield) was recovered: The melting point was 65—67°C.

TLC: Rf = 0.65 in toluene (silica gel plates).

Pink color developed when the product spot was irradiated with short UV light.

Analysis Calculated for: $C_{21}H_{30}O$: C, 84.51; H, 10.13.
Found: C, 84.49; H, 10.72.

Preparation of 7-(n-Decyl)-2-methyl-4-(3′,5′-dichlorophen-4′-one)-indonaphthol

7-Decylmethyl-1-naphthol (4.5 g, 15.1 mmoles) and 2,6-dichloroquinone-4-chloroimide (3.0 g, 14.3 mmoles) were dissolved in acetone (150 ml). The solution was treated with 700 ml potassium carbonate solution (0.1 M, pH=10.0). The solution was stirred vigorously at RT for 10 min. The pH of the reaction mixture was adjusted to 2.8 with HCl (1.0 μ). The mixture was stirred for 15 minutes. The red solid was recovered by filtration and washed well with water. The solid was dissolved in toluene and filtered with glass fiber paper to remove any insoluble materials. The filtrate was concentrated and purified with Prep-500 silica gel column, using toluene as the mobile phase. Product fractions were pooled and evaporated to dryness. The residue was crystallized with *n*-hexane (100 ml) to give product (3.9 g, 58% yield).

TLC: Rf = 0.26 in toluene (silica gel plates).

Brown color spot, turn purple-blue after treated with 0.1 N NaOH on the plates.

Analysis Calculated for: $C_{27}H_{31}NO_2Cl_2$: C, 68.64; H, 6.57; N, 2.97.
Found: C, 68.88; H, 6.85; N, 2.97.

This product, 7-decyl-MEDPIN, was used in the following experiments as the reporter substance.

2 Naphtho-15-crown-5 as Ionophore

An acetone mixture was prepared containing 10.8 mg (milligrams) 7-decyl-MEDPIN and 24 mg naphtho-15-crown-5 (2,3,5,6,8,9,11,12-octahydronaptho[2,3-b)-1,4,7,10,13-pentaoxacyclopentadecane). Solvent was removed under a stream of nitrogen gas. Then dried solids were combined with 4 g of a film solution comprising 70% by weight cyclohexanone, 12% by weight polyvinyl chloride/polyvinylidene copolymer, 18% by weight diethyl phthalate, and 60 (μl) Triton® X—100 (a 1% by weight solution of nonionic detergent in acetone; available from Rohm and Haas, Co.). The mixture was homogenized on a vortex mixer, and then spread into a thin film on a piece of KODAR® cyclohexanone/dimethylene terephthalate copolymer (Lustro Co.) channeled plastic, using a doctor blade having a 0,254 mm (10 mil (0.01 inch) gap. The dried film has a thickness of about 0,075 mm (3 mils).

The test means was evaluated with aqueous test samples containing various KCl concentrations. Each sample was 15.56 mM NaCl and 88.89 CAPS buffer [3-(cyclo-hexylamino)-propanesulfonic acid] and was adjusted to pH 10 with LiOH. The respective KCl concentrations were 0, 0.33, 0.67 and 1.0 mM.

The evaluations were conducted by inoculating a section of the test means film with 40 microliters (μl) of test sample and the change in reflectance monitored for one minute in a SERALYZER® reflectance Photometer (Ames Division of Miles Laboratories, Inc.)

The reflectance values (R) were converted to (K/S) in accordance with

$$(K/S) = \frac{(1-R)^2}{2R^2}$$

in which R is the fraction of reflectance from the test device, K is a constant, and S is the light scattering coefficient of the particular reflecting medium. The above equation is a simplified form of the well-known Kubelka-Munk equation (See Gustav Kortüm, "Reflectance Spectroscopy", pp. 106—111, Springer Verlag, New York (1969). The data is tabulated below as (K/S) with respect to time.

| $[K^+]$mM | (K/S) $second^{-1}$ |
|---|---|
| 0 | 0.001151 |
| 0.33 | 0.007679 |
| 0.67 | 0.01295 |
| 1.0 | 0.01909 |

As can be seen from the table, rate of change in (K/S) with time varies in accordance with potassium concentration. The data is shown graphically in Figure I, and demonstrates easy differentiation of various $K^+$ concentration levels.

3 Potassium Test Means Using Naphtho-15-crown-5 and Valinomycin as Ionophores

A test means film was prepared and evaluated as in Example 9.2 except that 6 mg of naphtho-15-crown-5 was replaced by 6 mg valinomycin. The data is reported in the following table:

| $[K^+]$mM | (K/S) $second^{-1}$ |
|---|---|
| 0 | 0.001182 |
| 0.33 | 0.007554 |
| 0.67 | 0.01331 |
| 1.0 | 0.01857 |

The data shows a direct correlation between potassium concentration and rate of change of (K/S), as is clearly depicted by the graphical depiction of the data in Figure II.

4 Potassium Test Means Using Equal Amounts of Naphtho-15-crown-5 and Valinomycin as Ionophores

A test means film was prepared and evaluated as in Example 9.2 except that 12 mg of naphtho-15-crown-5 was replaced by 12 mg valinomycin. The data is reported in the following table:

| $[K^+]$mM | (K/S) $second^{-1}$ |
|---|---|
| 0 | 0.0007449 |
| 0.33 | 0.008314 |
| 0.67 | 0.01251 |
| 1.0 | 0.01898 |

The data shows a direct correlation between potassium concentration and rate of change of (K/S). This is clearly shown by the graphical plot of the data in Figure III.

5 Potassium Test Means Using Valinomycin as Ionophore

A test means film was prepared and evaluatedas in Example 9.2 except that the amount of 7-decyl-MEDPIN was 5.4 mg, the ratio of polyvinyl chloride/polyvinylidene chloride copolymer to diethylphthalate was adjusted to 8.55:21.45 by weight, and the 12 mg naphtho-15-crown-5 was replaced by 12 mg valinomycin.

The aqueous test samples contained KCl at concentrations of 0, 0.33, 0.67, 1.0, 2.0 and 3.0 mM. In addition, each solution contained 46.67 mM NaCl, 66.67 mM CAPS and was titrated to pH 10 with LiOH.

The reflectance data is reported in the following table:

| $[K^+]$mM | (K/S) $second^{-1}$ |
|---|---|
| 0 | 0.001008 |
| 0.33 | 0.01090 |
| 0.67 | 0.01787 |
| 1.0 | 0.02872 |
| 2.0 | 0.04321 |
| 3.0 | 0.05330 |

As can be seen from the data, the test means exhibited a direct correlation between potassium concentration and the rate of change of (K/S) with time. As the plot of the data in Figure IV shows, easy differentiation between $K^+$ concentration levels was obtained.

6 Potassium Test Means Using Dipentyl Phthalate as Plasticizer

A test means film was prepared and evaluated as in Example 9.5 except that the diethyl phthalate was replaced by an equal weight of dipentyl phthalate.

Aqueous test samples were as in Example 9.5 and contained KCl as indicated in the table of data below:

| $[K^+]$mM | (K/S) $second^{-1}$ |
|---|---|
| 0 | 0.0005070 |
| 0.33 | 0.004041 |
| 0.67 | 0.007020 |
| 1.5 | 0.01391 |
| 3.0 | 0.02195 |

The data shows a direct correlation between potassium concentration and the rate of change of (K/S) with time. The data is plotted in Figure V, which portrays the ease of differentiation of various potassium levels using the test means film of the present example.

7 Sodium Test Means

A solution of 10.8 mg 7-decyl-MEDPIN in acetone and a solution of 5 mg sodium Ligand I [N,N',N''-triheptyl-N,N',N''-trimethyl-4,4',4''-propylidintris-3-oxa-butyramide] in tetrahydrofuran (THF) were mixed and the solvents removed under a stream of nitrogen. To the dried solids was added 0.5 g of a film solution. The latter was 70% by weight cyclohexanone, 8.55% by weight of vinylchloride/vinylidene chloride copolymer, and 21.45% by weight dipentyl phthalate. The mixture was homogenized on a vortex mixer and the homogenate spread into a film on a piece of KODAR channeled plastic using a 10 mil doctor blade. The dried film had a thickness of about 0,075 mm (3 mils).

Aqueous sodium test samples were prepared for evaluating the test means. Each contained 88.98 mM CAPS and KOH was added to adjust the pH to 10. Samples were prepared containing 11.11 mM and 22.22 mM NaCl, respectively.

To evaluate the ability of the test means to detect sodium, 40 µL of a test sample was applied to a section of the test means film and reflectance at 640 nm was monitored over 2 minutes in SERALYZER reflectance photometer. Refelectance values were converted to (K/S) values as in Examples 9.2. The rate of change of (K/S) with time and respective sodium concentrations are tabulated below:

| $[Na^+]$mM | (K/S) $second^{-1}$ |
|---|---|
| 0 | 0.001459 |
| 11.11 | 0.003148 |
| 22.22 | 0.004354 |

The data shows a direct correlation between sodium concentration and the rate of change of (K/S) with time, as portrayed graphically in Figure VI.

8 Tetrabomophenolphthalein Ethyl Ester Used as a Reporter Substance in Test Means for Detecting Potassium

A solution was prepared containing 1.8 mM valinomycin, 5 mM tetrabromophenolphthalein ethyl ester (TBEE), 5% by weight polyvinylchloride ("high molecular weight", Aldridge Chemical Co. Catalogue No. 18,956-1) and 13.1% by weight dipentyl phthalate in tetrahydrofuran. This solution was spread onto a polyester film using a 0,25 mm (10 mil) doctor blade, and dried.

The test means was evaluated using aqueous solutions (test samples) containing KCl at concentrations of 0, 0.222, 0.556 and 1.111 mM, respectively. Each solution contained 100 mM sodium citrate (pH = 5.3). The reflectance response of the test means to each test sample solution was monitored at 37°C and 520 nm using a SERALYZER reflectance spectrophotometer. The results are tabulated below.

| $[K^+]$mM | (K/S) $second^{-1} \times 10^{-3}$ |
|---|---|
| 0 | −0.0108 |
| 0.222 | 0.990 |
| 0.556 | 2.013 |
| 1.111 | 4.101 |

The data shows a direct correlation between rate of change of (K/S) per unit time and potassium concentration. Figure VII presents this data graphically, and demonstrates a linear relationship between actual $K^+$ concentration and (K/S).

**Claims**

1. A test means for determining the presence of an ion in an aqueous test sample, the test means comprising

a) a substantially nonpolar, nonporous carrier matrix which is incorporated with an ionophore capable of selectively forming a complex with the ion under analysis and

b) in addition, the carrier matrix is incorporated with a reporter substance which is capable of interacting with the complex of ionophore and ion to produce a detectable response.

2. A test means according to Claim 1, characterized in that the ionophore is a podand, a cryptand or a coronand and preferably is valinomycin 4,7,13,16,21-pentaoxa-1, 10-diazabicyclo[8,8,5]tricosane, 4,7,13,16,21,24-hexaoxa-1,10-diazabicyclo[8,8,8]hexacosane, 4,7,13,18-tetraoxa-1,10-diazabicyclo[8,5,5]-eicosane, 12-crown-4, 15-crosn-5, 18-crown-6, dibenzo-18-crown-6, or dicyclohexane-18-crown-6.

3. A test means according to Claim 1 or 2, characterized in that the reporter substance is a compound having the structure

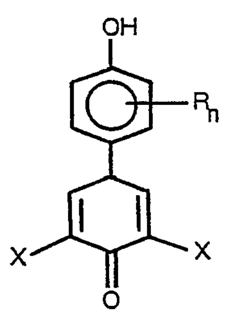

in which X is a halogen or pseudohalogen; R is a 2-, 3-, 5-, and/or 6-position substituent selected from $C_1$ to $C_{12}$ alkyl, aryl and a fused ring at the 2,3- or 5,6-positions; and n is 0 to 4.

4. A test means according to Claim 1 or 2, characterized in that the reporter substance is a compound having the structure

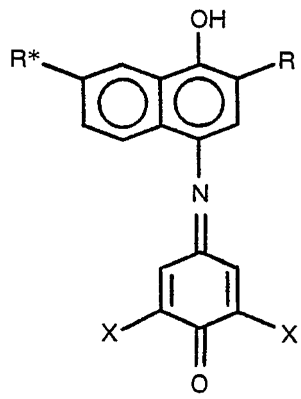

in which R is H or $C_1$ to $C_4$ alkyl, R* is H or $C_4$ to $C_{12}$ alkyl and X is a halogen or pseudohalogen.

5. A test means according to Claim 4, characterized in that R is methyl and R* is n-decyl.

6. A test means according to any of Claims 1 to 5, characterized in that the reporter substance is one capable of producing the appearance of, or change in, fluorescence in the presence of the complex of the ionophore and ion, and preferably is fluorescein or a derivative thereof.

7. A test means according to any of Claims 1 to 5, characterized in that the reporter substance comprises at least two precursor substances, which are capable of participating in the formation of a detectable response in the presence of the complex of the ionophore and the ion.

8. A test means according to Claim 7, characterized in that the precursor substances have the respective structure

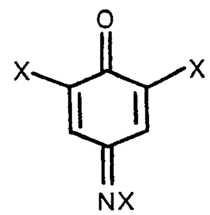

and

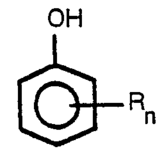

in which X is halogen or a pseudohalogen, R is a 2-, 3-, 5-, and/or 6-position substituent selected from $C_1$ to $C_{12}$ alkyl, aryl and a fused ring at the 2,3- or 5,6-positions, and n is 0—4.

9. A test means according to Claim 7, characterized in that the precursor substances are starch and an oxidizing agent.

10. A test for determining the presence of an ion in a test sample, the test device comprising

an elongated support member having an upper substantially flat face, and

the test means of any one of Claims 1—9 affixed to the flat face of the support member.

11. A method for determining the presence of an ion in an aqueous test sample, the method comprising contacting the test sample with the test means of any one of Claims 1—9 or the test device of Claim 10 and observing a detectable response.

**Patentansprüche**

1. Testmittel zur Bestimmung des Vorliegens eines Ions in einer wäßrigen Testprobe, wobei das Testmittel

a) eine im wesentlichen nicht-polare, nicht-poröse Trägermatrix umfaßt, die mit einem Ionophor inkorporiert ist, der befähigt ist, einen Komplex mit dem zu analysierenden Ion selektiv zu bilden, und wobei

b) die Trägermatrix zusätzlich mit einer Widergebesubstanz inkorporiert ist, die befähigt ist, mit dem Komplex aus Ionophor und Ion in Wechselwirkung zu treten, um eine nachweisbare Reaktion zu erzeugen.

2. Testmittel gemäß Anspruch 1, dadurch gekennzeichnet, daß der Ionophor ein Podand, Cryptand oder Coronand und vorzugsweise Valinomycin, 4,7,13,16,21-Pentaoxa-1,10-diazabicyclo[8,8,5]tricosan, 4,7,13,16,21,24-Hexaoxa-1,10-diazabicyclo[8,8,8]hexacosan, 4,7,13,18-Tetraoxa-1,10-diazabicyclo[8,5,5]-eicosan, 12-Crown-4, 15-Corwn-5, 18-Crown-6, Dibenzo-18-crown-6 oder Dicyclohexan-18-crown-6 ist.

3. Testmittel gemäß Anspruch 1 oder 2, dadurch gekennzeichnet, daß die Wiedergabesubstanz eine Verbindung mit der Struktur

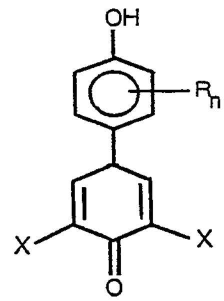

ist, worin X ein Halogen oder Pseudohalogen ist, R ein Substituent in 2-, 3-, 5- und/oder 6-Position ist, ausgewählt aus $C_1$ bis $C_{12}$-Alkyl, Aryl und einem kondensierten Ring an den 2-, 3- oder 5-, 6-Positionen, und n 0 bis 4 ist.

4. Testmittel gemäß Anspruch 1 oder 2, dadurch gekennzeichnet, daß die Wiedergabesubstanz eine Verbindung mit der Struktur

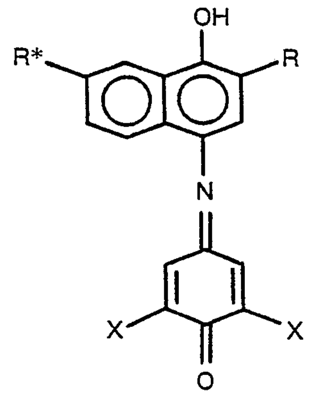

ist, worin R H oder $C_1$—$C_4$-Alkyl, R* H oder $C_4$-$C_{12}$-Alkyl und X ein Halogen oder Pseudohalogen sind.

5. Testmittel gemäß Anspruch 4, dadurch gekennzeichnet, daß R Methyl und R* n-Decyl sind.

6. Testmittel gemäß jedem der Ansprüche 1 bis 5, dadurch gekennzeichnet, daß die Wiedergabesubstanz befähigt ist, das Auftretren oder einen Wechsel der Fluoreszenz in der Gegenwart des Komplexes aus dem Ionophor und dem Ion zu erzeugen, und vorzugsweise Fluorescein oder ein Derivat davon ist.

7. Testmittel gemäß jedem der Ansprüche 1 bis 5, dadurch gekennzeichnet, daß die Wiedergabesubstanz mindestens zwei Vorläufersubstanzen umfaßt, die befähigt sind, an der Bildung einer nachweisbaren Reaktion in der Gegenwart des Komplexes aus dem Ionophor und dem Ion teilzunehmen.

8. Testmittel gemäß Anspruch 7, dadurch gekennzeichnet, daß die Vorläufersubstanzen die jeweilige Struktur

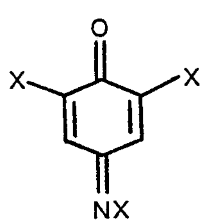

und

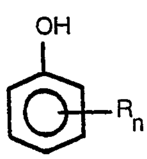

aufweisen, worin X Halogen oder Pseudohalogen, R ein Substituent in 2-, 3-, 5- und/oder 6-Position, ausgewählt aus $C_1$—$C_{12}$-Alkyl, Aryl und einem kondensierten Ring an den 2-, 3- oder 5-, 6-Positionen, und n 0 bis 4 sind.

9. Testmittel gemäß Anspruch 7, dadurch gekennzeichnet, daß die Vorläufersubstanzen Stärke und ein Oxidationsmittel sind.

10. Testvorrichtung zur Bestimmung des Vorliegens eines Ions in einer Testprobe, wobei die Testvorrichtung
einen verlängerten Unterlagenteil mit einem oberen im wesentlichen flachen Stück und
das Testmittel eines jeden der Ansprüche 1 bis 9, das auf dem Flachstück des Unterlagenteils angebracht ist,
umfaßt.

11. Verfahren zur Bestimmung des Vorliegens eines Ions in einer wäßrigen Testprobe, wobei das Verfahren umfaßt, daß man die Testprobe mit dem Testmittel eines jeden der Ansprüche 1 bis 9 oder der Testvorrichtung des Anspruchs 10 in Kontakt bringt und eine nachweisbare Reaktion beobachtet.

**Revendications**

1. Moyen pour test destiné à la détermination de la présence d'un ion dans un échantillon aqueux à tester, ce moyen pour test comprenant:

a) une matrice porteuse non-poreuse, substantiellement non-polaire, à laquelle est incorporé un ionophore capable de former sélectivement un complexe avec l'ion sous analyse, et

b) de plus, à la matrice porteuse est incorporé une substance indicatrice qui est capable d'entrer en interaction avec le complex d'ionophore et d'ion pour produire une réponse détectable.

2. Moyen pour test selon la revendication 1, caractérisé en ce que l'ionophore est un podand, un cyptand ou un coronand et est de préférence valinomycine, 4,7,13,16,21-pentaoxa-1,10-diazabicyclo[8,8,5]-tricosane, 4,7,13,16,21,24-hexaoxa-1,10-diazabicyclo[8,8,8]hexacosane, 4,7,13,18-tétraoxa-1,10-diazabicyclo[8,5,5]eicosane, 12-couronne-4, 15-couronne-5, 18-coronne-6, dibenzo-18-coronne-6, ou dicyclohexane-18-coronne-6.

3. Moyen pour test selon la revendication 1 ou 2, caractérisé en ce que la substance indicatrice est un composé de structure:

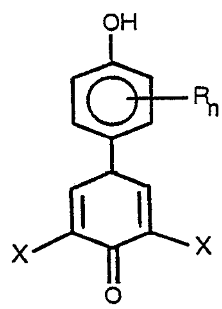

dans laquelle X est un halogène ou un pseudohalogène; R est un substituant à la position 2, 3, 5 et/ou 6 choisi parmi alkyle $C_1$ à $C_{12}$, aryle et un noyau condense aux positions 2, 3 ou 5, 6; et n est 0 à 4.

4. Moyen pour test selon la revendication 1 ou 2, caractérisé en ce que la substance indicatrice est un composé de structure:

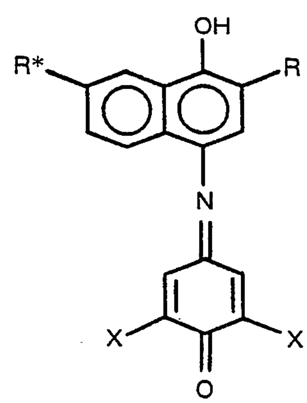

dans laquelle R est H ou alkyle $C_1$ à $C_4$, R* est H ou alkyle $C_4$ à $C_{12}$ et X est un halogène ou un pseudohalogène.

5. Moyen pour test selon la revendication 4, caractérisé en ce que R est méthyle et R* est n-décyle.

6. Moyen pour test selon n'importe laquelle des revendications 1 à 5, caractérisé en ce que la substance indicatrice est une substance capable de produire l'apparition ou un changement de fluorescence en présence du complexe de l'ionophore et de l'ion, et est de préférence la fluorescéine ou un de ses dérivés.

7. Moyen pour test selon n'importe laquelle des revendications 1 à 5, caractérisé en ce que la substance indicatrice comprend au moins deux substances précurseurs qui sont capables de participer à la formation d'une réponse détectable en présence du complexe de l'ionophore et de l'ion.

8. Moyen pour test selon la revendication 7, caractérisé en ce que les substances précurseurs ont les structures respectives:

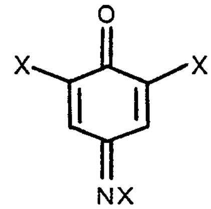

et

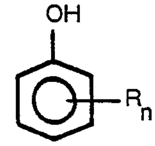

dans lesquelles X est un halogène ou un pseudohalogène, R est un substituant à la position 2, 3, 5 et/ou 6 choisi parmi alkyle $C_1$ à $C_{12}$, aryle et un noyau condensé aux positions 2, 3 ou 5, 6, et n est 0 à 4.

9. Moyen pour test selon la revendication 7, caractérisé en ce que les substances précurseurs sont l'amidon et un agent oxydant.

10. Dispositif pour test destiné à la détermination de la présence d'un ion dans un échantillon à tester, ce dispositif pour test comprenant:

un membre support allongé ayant une face supérieure substantiallement plane, et

le moyen pour test selon n'importe laquelle des revendications 1 à 9 fixé sur la surface plane due membre support.

11. Procédé pour la détermination de la présence d'un ion dans un échantillon aqueux à tester, ce procédé comprenant la mise en contact de l'échantillon à tester avec le moyen pour test selon n'importe laquelle des revendications 1 à 9 ou le dispositif pour test selon la revendication 10, et l'observation d'une réponse détectable.

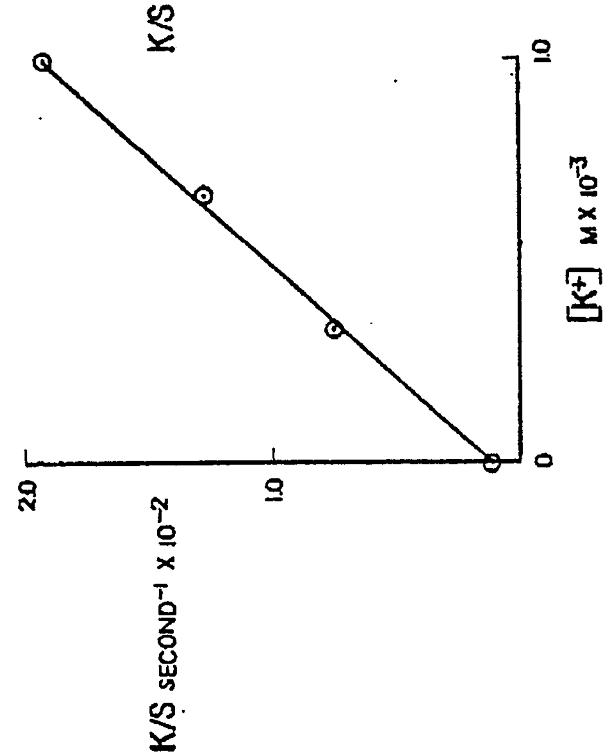
K/S SECOND$^{-1}$ X 10$^{-2}$
2.0
1.0
0
[K+] M X 10$^{-3}$
1.0

FIG. 1

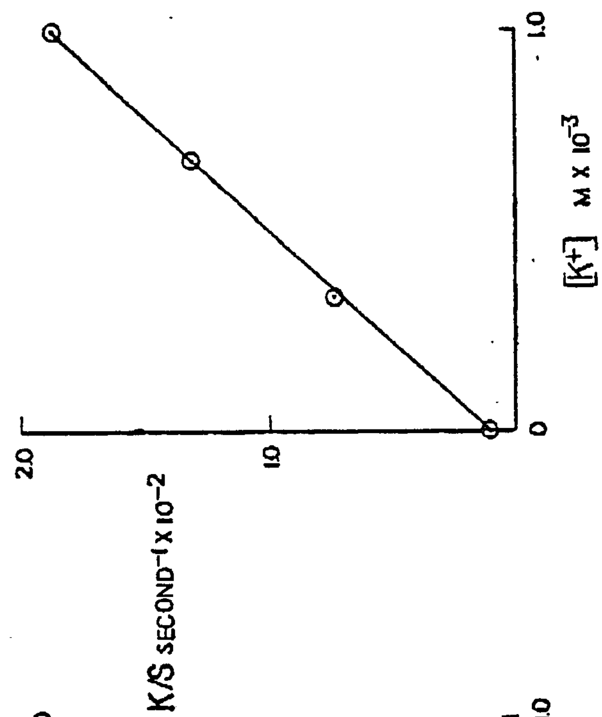
K/S SECOND$^{-1}$ X 10$^{-2}$
2.0
1.0
0
[K+] M X 10$^{-3}$
1.0

FIG. 2

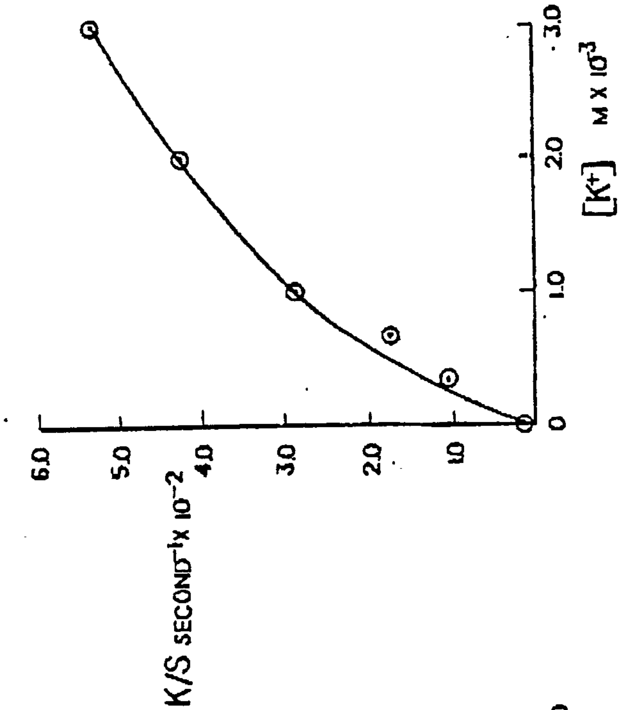
K/S SECOND$^{-1}$x 10$^{-2}$
6.0
5.0
4.0
3.0
2.0
1.0
0
1.0
2.0
3.0
[K$^{+}$] M x 10$^{-3}$

FIG. 4

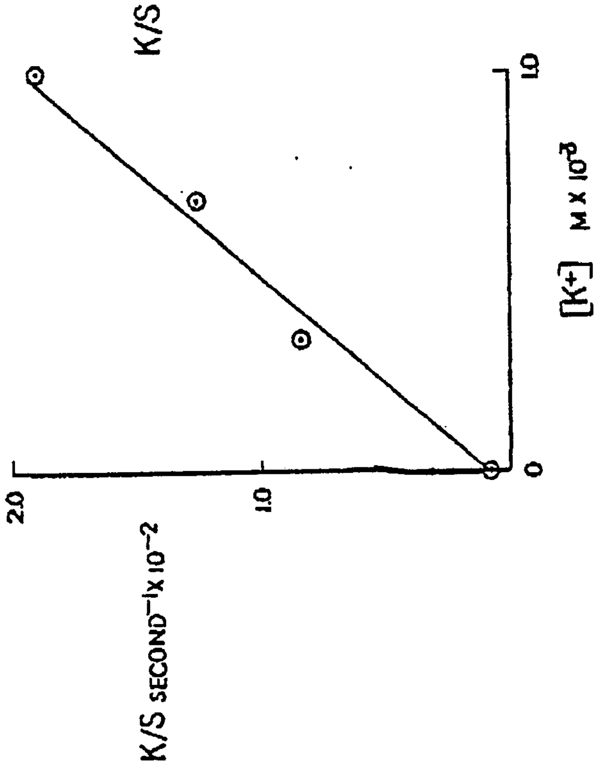
K/S SECOND$^{-1}$x 10$^{-2}$
2.0
1.0
0
1.0
[K$^{+}$] M x 10$^{-3}$

FIG. 3

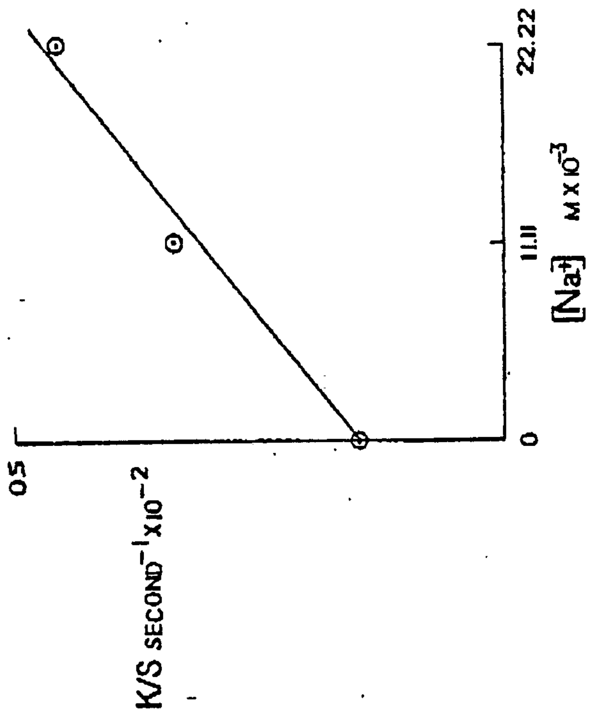
K/S SECOND$^{-1}$ x10$^{-2}$
0.5
0
11.11
22.22
[Na$^{+}$] M x 10$^{-3}$

FIG. 6

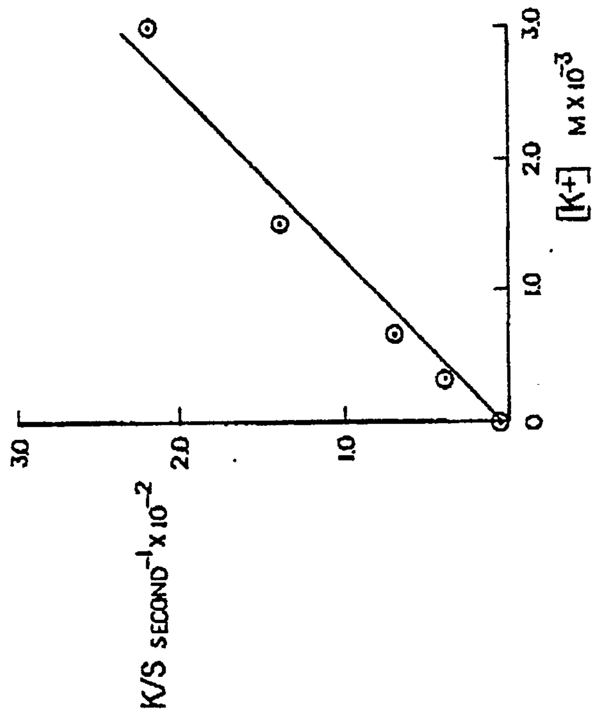
K/S SECOND$^{-1}$ x 10$^{-2}$
3.0
2.0
1.0
0
1.0
2.0
3.0
[K$^{+}$] M x 10$^{-3}$

FIG. 5

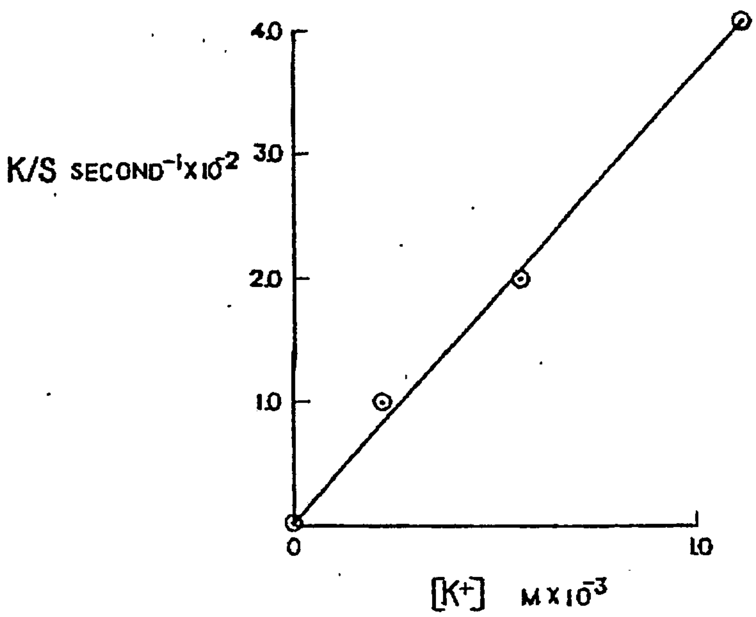


FIG. 7